# EUROPEAN PATENT SPECIFICATION

(11) **EP 3 176 753 B1**
(45) Date of publication and mention of the grant of the patent: **24.10.2018**
(21) Application number: 16203990.3
(22) Date of filing: 02.04.2015
(51) Int. Cl.: G06T 7/62, G06T 7/00, G06T 7/73

(54) **IMAGE ANALYSIS IN THE PRESENCE OF A MEDICAL DEVICE**
BILDANALYSE IN GEGENWART EINER MEDIZINISCHEN VORRICHTUNG
ANALYSE D'IMAGE EN PRÉSENCE D'UN DISPOSITIF MÉDICAL

(30) Priority: 10.04.2014 US 201461977891 P
(43) Date of publication of application: 07.06.2017
(62) Divisional of application: 15776785.6
(73) Proprietor: Sync-RX, Ltd., 42505 Netanya (IL)
(72) Inventor: KLAIMAN, Eldad, 5656 AE Eindhoven (NL); STEINBERG, Alexander, 5656 AE Eindhoven (NL); KARMON, Nili, 5656 AE Eindhoven (NL); SEMO, Sarit, 5656 AE Eindhoven (NL); COHEN, Ran, 5656 AE Eindhoven (NL)
(74) Representative: de Haan, Poul Erik

(56) References cited:
- WO-A1-2013/169814
- WO-A2-2009/024894
- E M Franken: "Context-enhanced detection of electrophysiology catheters in noisy fluoroscopy images", Master Thesis of Eindhoven University of Technology, 1 January 2004 (2004-01-01), XP055357542, Retrieved from the Internet: URL:https://pure.tue.nl/ws/files/46971957/ 601376-1.pdf [retrieved on 2017-03-22]
- T. LINDEBERG: "Edge detection and ridge detection with automatic scale selection", PROCEEDINGS CVPR IEEE COMPUTER SOCIETY CONFERENCE ON COMPUTER VISION AND PATTERN RECOGNITION, vol. 30, 1 January 1996 (1996-01-01), pages 465-470, XP055103382, DOI: 10.1109/CVPR.1996.517113 ISBN: 978-0-81-867258-3

## Description

### CROSS REFERENCES TO RELATED APPLICATIONS

The present application claims priority from US Provisional Patent Application 61/977,891 to Klaiman, filed April 10, 2014, entitled "Image analysis in the presence of a medical device,".

### FIELD OF EMBODIMENTS OF THE INVENTION

Some applications of the present invention generally relate to medical imaging. Specifically, some applications of the present invention relate to medical imaging and analysis of such images when such images are acquired in the presence of a tool in the subject's body.

### BACKGROUND

Vascular catheterizations, such as coronary catheterizations, are frequently-performed medical interventions. Such interventions are typically performed in order to diagnose the blood vessels for potential disease, and/or to treat diseased blood vessels. Typically, in order to enable observation of blood vessels, the catheterization is performed under extraluminal imaging. Additionally, for some procedures, an endoluminal data-acquisition device is used to perform endoluminal imaging and/or measurements. The extraluminal imaging and, where applicable, the endoluminal data are typically evaluated by the medical staff in combination with one another in the course of the intervention, as well as post procedurally.

The Master Thesis "Context-enhanced detection of electrophysiology catheters in noisy fluoroscopy images" by E.M. Franken, 2004, discloses a method for detecting the endpoint of a guidewire. A ridgeness measure is derived from the Hessian. A combination of the ridgeness with the intensity gradient provides an "end-pointness" measure used in a tensor voting to detect the tip of a guide wire (e.g. section C.1.2 in p.125).

### SUMMARY OF EMBODIMENTS

An apparatus according to the invention is defined in claim 1.

For some applications of the present invention, the location of a tip of a radiopaque portion of a wire within an image of a blood vessel is automatically determined by a computer processor. Typically, for each of the pixels within at least a portion of the image, a wireness measure (i.e., a measure of the extent to which the pixels has a wire-like characteristic) is determined by the computer processor, by measuring an extent to which the pixel, together with other pixels within the image, forms part of a long, thin set of pixels having a given characteristic, such as darkness, brightness, and/or a different characteristic (i.e., a wireness-indicating characteristic). In addition, for each of the pixels within at least the portion of the image the pixel intensity is determined by the computer processor. Subsequently, it is determined by the computer processor whether there is at least one pixel at which there is a change in the wireness measure by more than a threshold amount, relative to at least some of the pixels that belong to the set of pixels that have the wireness-indicating characteristic. In response to determining that the change in the wireness measure of at least one pixel within a given sampling region does exceed the threshold, the computer processor then determines whether at the at least one pixel there is a change, by more than a threshold amount, in the intensity of the pixel relative to the value of the intensity of at least some of the set of pixels that have the wireness-indicating characteristic. In response to determining that the change in intensity at the at least one pixel within the given sampling region does exceed the threshold, it is determined by the computer processor that the tip of the radiopaque portion of the wire is disposed within the given sampling region.

For some applications, within the designated sampling regions, the local directionality of the set of pixels that have the wireness-indicating characteristic is determined, and the changes in the wireness measure and/or intensity are measured along that direction. For some applications, it is first determined whether at at least one pixel within the sampling regions there is a change in intensity that exceeds the threshold, and, in response to determining that at at least one pixel within a given sampling region there is a change in intensity that exceeds the threshold, it is determined whether at the at least one pixel there is a change in the wireness measure that exceeds the threshold.

For some applications, in response to determining the location of the tip of the radiopaque portion of the wire within a given image, the image is aligned with a second image, by aligning the radiopaque portions of the guidewires in each of the images with one another. Alternatively or additionally, identification of the location of the tip of the radiopaque portion of the wire within a given image is used to facilitate the determination of the location of an endoluminal device within the lumen, for example, in accordance with techniques described in US 2012/0004537 to Tolkowsky, and/or WO 13/174472 to Steinberg. Further alternatively or additionally, identification of the location of the tip of the radiopaque portion of the wire within a given image is used to facilitate the determination of a transformation function for mapping between the image and a second image, for example, in accordance with techniques described in WO 13/174472 to Steinberg.

The present invention will be more fully understood from the following detailed description of embodiments thereof, taken together with the drawings, in which:

### BRIEF DESCRIPTION OF THE DRAWINGS

Fig. 1 is a schematic illustration of apparatus this used in a catheterization laboratory, in accordance with some applications of the present invention;
Fig. 2A is a schematic illustration of an extraluminal image of a blood vessel that has lesion, in accordance with some applications of the present invention;
Fig. 2B is a schematic illustration of an extraluminal image of an angioplasty balloon that has been maximally inflated inside a blood vessel, such as to treat an occlusion, in accordance with some applications of the present invention;
Fig. 2C-D are extraluminal images of an angioplasty balloon that has been maximally inflated inside a blood vessel, such as to treat an occlusion, in accordance with some applications of the present invention;
Figs. 3A-C are flowcharts showing steps of a procedure that is performed by a processor in order to designate a parameter to be calculated, in accordance with some applications of the present invention;
Fig. 4 is a schematic illustration of an image of a blood vessel, a wire (e.g., a guidewire) being disposed inside the blood vessel, in accordance with some applications of the present invention; and
Fig. 5 is a flowchart showing steps of a procedure that is performed by a processor in order to determine a location of a tip of a radiopaque portion of a wire within an extraluminal image of a blood vessel, in accordance with some applications of the present invention.

### DETAILED DESCRIPTION OF EMBODIMENTS

- The terms "medical tool," "tool", "device," and "probe" refer to any type of a diagnostic or therapeutic or other functional tool including, but not limited to, a cardiovascular catheter, a stent delivery, placement and/or retrieval tool, a balloon delivery and/or placement and/or retrieval tool, a valve delivery and/or repair and/or placement and/or retrieval tool, a graft delivery and/or placement and/or retrieval tool, a tool for the delivery and/or placement and/or retrieval of an implantable device or of parts of such device, an implantable device or parts thereof, a tool for closing a gap, a tool for closing a septal defect, a guide wire, a marker wire, a suturing tool, a clipping tool (such as a valve-leaflet-clipping tool), a biopsy tool, an aspiration tool, a navigational tool, a localization tool, a probe comprising one or more location sensors, a tissue characterization probe, a probe for the analysis of fluid, a measurement probe, an electrophysiological probe, a stimulation probe, an ablation tool, a tool for penetrating or opening partial or total occlusions in blood vessels, a drug or substance delivery tool, a chemotherapy tool, a photodynamic therapy tool, a brachytherapy tool, a local irradiation tool, a laser device, a tool for delivering energy, a tool for delivering markers or biomarkers, a tool for delivering biological glue, an irrigation device, a suction device, a ventilation device, a device for delivering and/or placing and/or retrieving a lead of an electrophysiological device, a lead of an electrophysiological device, a pacing device, a coronary sinus device, an imaging device, a sensing probe, a probe comprising an optical fiber, a robotic tool, a tool that is controlled remotely, an excision tool, a plaque excision tool (such as a plaque excision catheter), or any combination thereof.
- The terms "image" and "imaging" refer to any type of medical images or imaging, typically resulting in the generation of a sequence of images and including, but not limited to, imaging using ionizing radiation, imaging using non-ionizing radiation, video, fluoroscopy, angiography, ultrasound, CT, MR, PET, PET-CT, CT angiography, SPECT, Gamma camera imaging, Optical Coherence Tomography (OCT), Near-Infra-Red Spectroscopy (NIRS), Vibration Response Imaging (VRI), optical imaging, infrared imaging, electrical mapping imaging, other forms of functional imaging, Focused Acoustic Computed Tomography (FACT), Optical Frequency Domain Imaging (OFDI), or any combination or fusion thereof. Examples of ultrasound imaging include Endo-Bronchial Ultrasound (EBUS), Trans-Thoracic Echo (TTE), Trans-Esophageal Echo (TEE), Intra-Vascular Ultrasound (IVUS), Intra-Cardiac Ultrasound (ICE), or any combination thereof.

- The term "contrast agent," when used in reference to its application in conjunction with imaging, refers to any substance that is used to highlight, and/or enhance in another manner, the anatomical structure, functioning, and/or composition of a bodily organ while the organ is being imaged.
- The term "stabilized," when used in the context of displayed images, means a display of a series of images in a manner such that periodic, cyclical, and/or other motion of the body organ(s) being imaged, and/or of a medical tool being observed, is partially or fully reduced, with respect to the entire image frame, or at least a portion thereof.
- The term "automatic," when used for describing the generation and utilization of the roadmap, means "without necessitating user intervention or interaction." (Such interaction or intervention may still however be optional in some cases.)
- The term "real-time" means without a noticeable delay.
- The term "near real-time" means with a short noticeable delay (such as approximately one or two motion cycles of the applicable organ, and, in the case of procedures relating to organs or vessels the motion of which are primarily a result of the cardiac cycle, less than two seconds).
- The term "on-line," when used in reference to image processing, or to measurements being made on images, means that the image processing is performed, and/or the measurements are made, intra-procedurally, in real-time or near real-time.

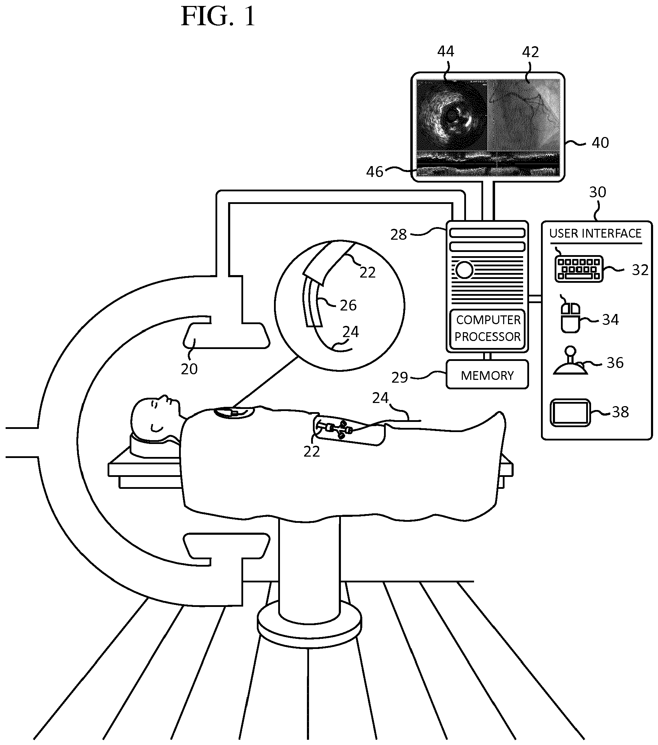

Reference is now made to Fig. 1, which is a schematic illustration of apparatus this used in a catheterization laboratory, in accordance with some applications of the present invention. Typically, a subject is imaged using an extraluminal imaging device (i.e., an extraluminal image-acquisition device) 20, which may include a fluoroscope that acquires fluoroscopic images under regular mode and/or under angiographic mode, while there is a presence of contrast agent in the blood vessels of the subject that are being imaged. For some applications, the imaging device performs fluoroscopy, CT, MR, PET, SPECT, ultrasound, or any combination thereof.

Fig. 1 additionally shows a guide catheter 22 that has been inserted into blood vessels of the subject (e.g., coronary arteries of the subject) over a guidewire 24. An endoluminal medical device 26 has been inserted into a blood vessel of the subject (e.g., into a coronary artery of the subject) through the guide catheter and over the guidewire. A computer processor 28 typically receives inputs from the imaging device. The computer processor communicates with a memory 29. Via a user interface 30, a user (e.g., a physician and/or a catheterization laboratory technician) sends instructions to the computer processor. For some applications, the user interface includes a keyboard 32, a mouse 34, a joystick 36, a touchscreen device 38 (such as a smartphone or a tablet computer), a touchpad, a trackball, a voice-command interface, and/or other types of user interfaces that are known in the art. Typically, the computer processor generates an output using an output device 40. Further typically, the output device includes a display, such as a monitor (as shown in Fig. 1), and the output includes an output that is displayed on the display. For some applications, the display includes a head-up display and/or a head-mounted display, such as Google Glass®. For some applications, the processor generates an output on a different type of visual, text, graphics, tactile, audio, and/or video output device, e.g., speakers, headphones, a smartphone, or a tablet computer. For some applications, user interface 30 acts as both an input device and an output device. For some applications, the processor generates an output on a computer-readable medium, such as a disk, or a portable USB drive.

It is noted that, for some applications, more than one processor is used. For some applications, more than one extraluminal imaging device is used with processor 20. For example, a first extraluminal imaging device may be used to acquire a first set of extraluminal images, and a second extraluminal imaging device maybe used to acquire a second set of extraluminal images.

For some applications, endoluminal medical device 26 includes an endoluminal data-acquisition device that is configured to acquire data (e.g., functional data or images) from inside the subject's blood vessels. For some applications, the endoluminal data-acquisition device is an imaging probe. For some applications, the imaging probe is an IVUS probe, an EBUS probe, a different type of ultrasound probe, an OCT probe, an NIRS probe, an MR probe, a FACT probe, an OFDI probe, or any combination thereof. For some applications, the endoluminal data-acquisition device performs additional functions. For example, the endoluminal data-acquisition device may comprise a probe, such as the VIBE(TM) RX Vascular Imaging Balloon Catheter, marketed by Volcano Corporation (San Diego, USA), that includes both IVUS and coronary balloon functionalities. For some applications, the endoluminal data-acquisition device acquires data in a form other than images. For example, the data may include data related to pressure, flow, temperature, electrical activity, oxygenation, biochemical composition, or any combination thereof. For some applications, and typically when data are acquired with respect to a coronary vessel, the endoluminal data-acquisition device is a Fractional Flow Reserve (FFR) probe, and/or an instantaneous wave-free ratio (iFR) probe. For some applications, FFR and/or iFR measurements are determined by performing image-processing on extraluminal images, and the derived FFR and/or iFR measurements are co-registered with endoluminal images of the lumen, using techniques described herein. For some applications, FFR and/or iFR measurements are determined by performing image-processing on endoluminal images, and the derived FFR and/or iFR measurements are co-registered with extraluminal images of the lumen, using techniques described herein. For some applications, endoluminal images are co-registered with extraluminal images of the lumen, using techniques described herein, and FFR and/or iFR measurements are determined by performing image-processing on the co-registered images.

For some applications, endoluminal medical device 26 includes an endoluminal therapeutic device that is positioned and/or deployed at an anatomical feature that requires or potentially requires treatment, such as a partial or total occlusion, a native valve, an aneurism, a dissection, a malformation, a septal defect, a mass suspected of being malignant, a mass suspected of being inflammatory, etc. For example, the endoluminal therapeutic device may include a balloon (e.g., an angioplasty balloon), a stent, a valve, and/or a wire (e.g., a guide wire).

For some applications, apparatus and methods described herein are used with an endoluminal therapeutic device that is positioned and/or deployed at an implantation site of a previously-implanted device such as a stent, a graft or a replacement valve. The endoluminal data are determined at, and/or in the vicinity of, the implantation site. For example, the techniques described herein may be used during the placement of a new prosthetic aortic valve at the site of (e.g., inside) a previously implanted prosthetic aortic valve that is no longer functioning.

For some applications, apparatus and methods described herein are used with an endoluminal therapeutic device that is positioned and/or deployed at a defined location relative to a previously-implanted device such as a stent, a graft or a replacement valve. The endoluminal data are determined at, and in the vicinity of, the defined location. For example, the techniques described herein may be used during the placement of a coronary stent such that the new stent overlaps with or is adjacent to a previously-implanted stent, in order to treat a long lesion and/or a lesion that has diffused along a coronary artery.

For some applications, output device 40 is a display that is configured to display an extraluminal image 42 of a blood vessel (e.g., a fluoroscopic image), an endoluminal image of a blood vessel 44 (e.g., an IVUS image), and or a stack 46 of cross-sections of endoluminal images (e.g., a stack of IVUS images).

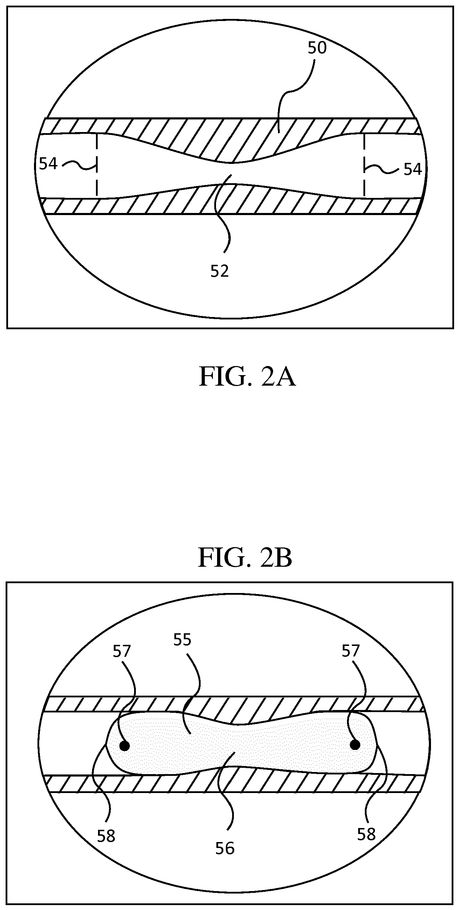

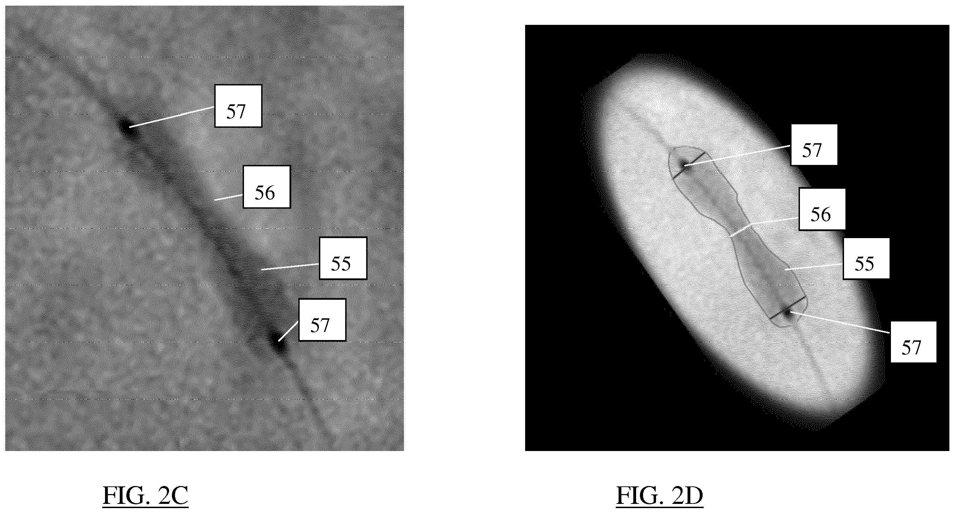

Reference is now made to Figs. 2A and 2B, which are schematic illustrations of extraluminal images of a blood vessel, in accordance with some applications of the present invention. Reference is also made to Figs. 2C and 2D, which are images of a balloon disposed inside an artery, in accordance with some applications of the present invention. Fig. 2D shows an enhanced version of the image shown in Fig. 2C, with the edge lines of the balloon marked upon the image.

Fig. 2A is a schematic illustration of an extraluminal image of a blood vessel 50 (such as a coronary artery) that has lesion, e.g., a partial occlusion 52, in accordance with some applications of the present invention. Typically, in the absence of a tool inside the vessel, in response to a user indicating a location of the lesion (e.g., by the user indicating a single point in the vicinity of the lesion in the image), the processor automatically performs quantitative vessel analysis on the blood vessel in the vicinity of the lesion. Typically techniques such as those described in US 2012/0230565 to Steinberg, and/or US 2010/0222671 to Cohen, are used for performing quantitative vessel analysis in the vicinity of the lesion. For example, using an input device (e.g., user interface 30), the user may designate the location (for example, by clicking a single click or a plurality of clicks at or near the location using the input device), and in response to the user designating the location, the system automatically detects a lesion in the vicinity. For example, the system may identify edge lines and the reference diameters 54 of the lesion. The reference diameters of a lesion are typically the diameters of the vessel at the longitudinal extremities of the lesion (the longitudinal extremities also being known as "healthy shoulders," or "reference arteries" to those skilled in the art). For some applications, the reference diameters are the broadest location within the section of the blood vessel that is analyzed. In response to detecting the lesion, quantitative vessel analysis is performed with respect to the lesion. For some applications, the lesion is graphically indicated, for example, by highlighting or coloring the section of the vessel that is determined to be the lesion. For some applications, measurements such as lesion length, the diameter of the vessel at each point along the centerline, and/or minimum lumen diameter is determined in the vicinity of the lesion. For some applications, the level of occlusion (which is typically provided as a percentage) at the minimum lumen diameter is determined by comparing the diameter of the vessel at that point, to the diameter of the vessel at reference points of the vessel.

Typically, the quantitative vessel analysis is performed by determining the locations of vessel centerlines and/or edge lines, for example, using techniques such as those described in US 2012/0230565 to Steinberg, and/or US 2010/0222671 to Cohen. For some applications, a lesion is automatically detected in accordance with the following procedure.

Scan lines are generated perpendicular to the centerline of a segment of the vessel that is sampled. The image is resampled along the scan lines. Corresponding gray-level values are stored as columns of a rectangular matrix M, thereby resampling the segment of the vessel as a straightened vessel segment. For the straightened vessel segment, optimum upper and lower paths are determined (with respect to the middle row of M), which connect the first and last columns of M. The optimization criterion takes into account the changes in gray-level along columns of M, and the paths' slopes. The vessel edge lines are obtained via back projection of upper and lower optimal paths on the original image.

A shortest path algorithm (e.g., as described in an article by Dijkstra, entitled "A Note on Two Problems in Connexion with Graphs" (Numerische Mathematik 1, 269-271, 1959)) is used in order to avoid irregularities, such as small gaps and loops, in the edge lines. For some applications, the centerline is corrected based upon the detected edge lines, and new scan lines are constructed. For each new scan line, vessel diameter is defined as a distance between the two points where the scan line intersects vessel boundaries.

Fig. 2B is a schematic illustration of an extraluminal image of an angioplasty balloon 55 (e.g., a compliant angioplasty balloon) that has been maximally inflated inside the blood vessel (i.e., inflated to the maximum pressure to which the balloon can safely be inflated in such as vessel), such as to treat the occlusion, in accordance with some applications of the present invention. Figs. 2C and 2D are actual images of angioplasty balloon 55, the balloon having been inflated inside an artery at a partial occlusion, in accordance with some applications of the present invention. Fig. 2D shows an enhanced version of the image shown in Fig. 2C, with the edge lines of the balloon marked upon the image. As shown in Figs. 2B-D, in some cases, even after the balloon is maximally inflated, the occlusion is not fully treated, but maintains what is known as a residual waist 56. It is typically desirable to be able to calculate the diameter of the vessel at the residual waist of the occlusion.

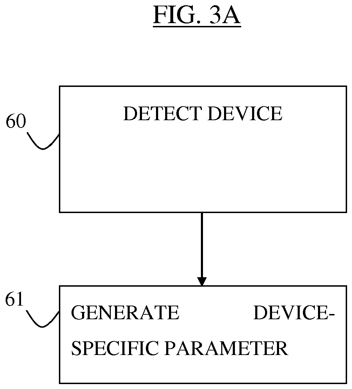

For some applications, if the processor uses the algorithm described hereinabove for performing quantitative vessel analysis on the vessel with the balloon inside, the processor may identify one or both ends 58 of the balloon as being the location of the minimal lumen diameter, since the system may not differentiate between edge lines of the vessel and edge lines of the balloon. Therefore, for some applications, in order to avoid the processor identifying one or both ends 58 of the balloon as being the location of the minimal lumen diameter, the processor determines that the balloon is present within the blood vessel. The processor determines a parameter of the vessel responsively to determining the presence of the balloon within the vessel, for example, in accordance with the techniques described hereinbelow with reference to Figs. 3A-C.

Reference is now made to Figs. 3A-C, which are flowcharts showing steps of a procedure that is performed by computer processor 28, in accordance with some applications of the present invention.

As shown in Fig. 3A, for some applications, the processor detects a device (step 60). Typically, the processor detects the device within an image of a portion of the subject's body, as described hereinbelow. In response to detecting the device, the processor generates as an output (e.g., on output device 40) a device-specific parameter (step 61), e.g., using the techniques described hereinbelow.

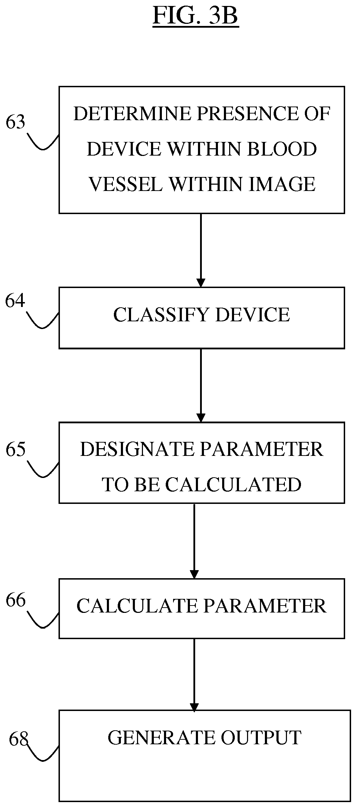

As shown in Fig. 3B, for some applications, the processor determines a presence of a device within a blood vessel within an image (step 63), and classifies the device as a given type of device (step 64), such as a balloon. In response to determining the classification of the device, the processor designates a parameter to be calculated (step 65). It is noted that, for some applications, the determination of the presence of a device within a blood vessel within an image (step 63), and the classification of the device as a given type of device (step 64) are performed simultaneously, or in the reverse order to that shown in Fig. 3B. For some applications, the user indicates that a given type of device (e.g., a balloon) is currently being inserted into the blood vessel (or is going to be inserted, or has been inserted, into the blood vessel), via user interface 30. Based upon the indication from the user, the processor automatically determines when the device is present inside the blood vessel, and the proceeds to step 65.

Subsequent to designating the parameter to be calculated (step 65), the processor calculates the designated parameter (step 66) and generates an output in response thereto (step 68). For example, in the example of angioplasty balloon 55, shown in Fig. 2B, in response to classifying the device as a balloon, the processor may designate as the parameter to be calculated, the minimum diameter of the vessel between the two ends of the balloon, and/or between two radiopaque markers 57 (Fig. 2B) of the balloon, which corresponds to the residual waist of the occlusion.

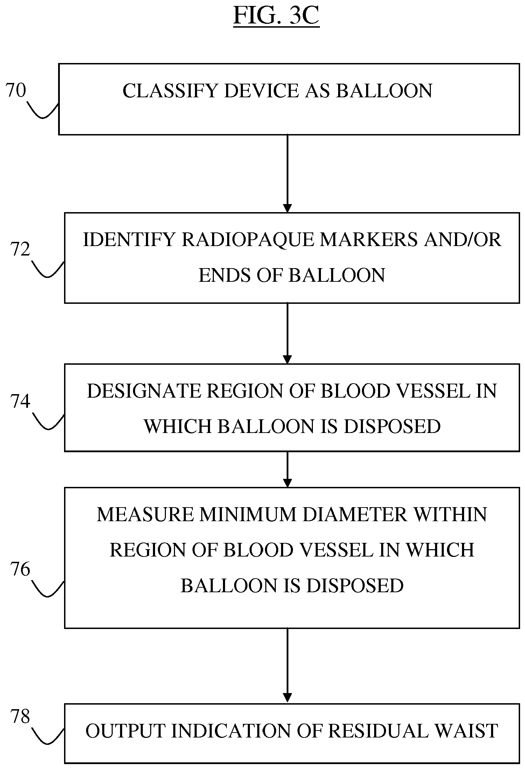

As shown in Fig. 3C, for some applications, in order to calculate the residual waist of the occlusion, in response to classifying the device as a balloon (step 70), the processor identifies locations of ends of the balloon and/or locations of radiopaque balloon markers 57 (step 72). Typically, the processor identifies balloon markers using image processing techniques, e.g., using techniques described herein for identifying balloon markers, and/or using techniques as described in US 2012/0230565 to Steinberg, and/or US 2010/0222671 to Cohen. For some applications, the processor determines locations of ends of the balloon, e.g., by detecting a location within the image in which there are generally straight edge lines (corresponding to the vessel edge lines), and within the straight edge lines there are tapered pairs of edge lines (corresponding to the tapered edges of the balloon).

The processor designates as a region of the vessel in which the balloon is disposed, a region of the vessel that is between the radiopaque markers of the balloon, and/or a region of the vessel that is between the tapered pairs of edge lines at each longitudinal end of the balloon (step 74). The processor then determines the minimum lumen diameter within the region of the vessel in which the balloon is disposed (step 76). The minimum lumen diameter within the region of the vessel in which the balloon is disposed is the residual waist of the occlusion. The processor then generates an output that is indicative of the calculated residual waist (step 78).

For some applications, the detection and/or classification of the device (steps 63 and 64 of Fig. 3B) are performed automatically by the processor using one or more algorithms described herein. For example, the processor may use automatic image-processing techniques to determine the presence of and/or the classification of the device. For some applications, the processor uses a machine-learning algorithm in order to automatically classify the device. For such applications, the processor compares an appearance of and/or characteristics of the detected device to machine-learnt appearances and characteristics. Alternatively or additionally, the processor compares an appearance of and/or characteristics of a region of the image to machine-learnt characteristics and appearances. Further alternatively or additionally, the processor receives an input from a user (typically via user interface 30) that is indicative of the presence of the device inside the vessel, and/or the classification of the device. As described hereinabove, for some applications, the user indicates that a given type of device (e.g., a balloon) is currently being inserted into the blood vessel (or is going to be inserted, or has been inserted, into the blood vessel), via user interface 30. Based upon the indication from the user, the processor automatically determines when the device is present inside the blood vessel, and then proceeds to step 65.

For some applications, the processor designates the parameter to be calculated (step 65 of Fig. 3B), using one or more algorithms described herein. For some applications, the processor designates the parameter to be calculated by designating a parameter of the blood vessel to be calculated. In accordance with some applications, the parameter of the blood vessel is a dimension of the blood vessel and/or a functional parameter of the blood vessel. For example, in response to classifying the device as a stent, the processor may designate, as the parameter to be calculated, the minimum lumen diameter of the blood vessel within a region of the blood vessel in which the stent is disposed, in order to determine the minimum lumen diameter of the vessel in presence of the stent. For some applications, the stent is disposed around a balloon, and the processor determines the region of the blood vessel in which the stent is disposed by determining locations of the radiopaque markers of the balloon around which the stent is disposed.

Alternatively or additionally, in response to classifying the device as a stent, the processor may designate functional flow reserve (or another luminal flow-related index) at the location of the stent as the parameter to be calculated, in order to determine the effect of the stent on the functional flow reserve (or the other luminal flow-related index) of the vessel. For some applications, the processor designates the parameter to be calculated by designating a parameter of the device to be calculated. For example, in response to classifying the device as a stent, the processor may designate a maximum diameter of the stent, or a minimum diameter of the stent as the parameter to be calculated. For some applications, the stent is disposed around a balloon, and the processor determines the region of the blood vessel in which the stent is disposed by determining locations of the radiopaque markers of the balloon around which the stent is disposed.

For some applications, the processor designates an event and designates the parameter to be calculated by designating a parameter to be calculated at the occurrence of the event. For example, in the example described with reference to Fig. 2B and 3C, the processor may designate maximum inflation of the balloon as the event, and the processor may determine the residual waist of the occlusion at the occurrence of maximal balloon inflation. For some applications, the processor automatically detects the occurrence of the designated event, e.g., using automatic image-processing techniques.

Typically, the parameter is calculated (step 66 of Fig. 3B), using one or more algorithms described herein. For some applications, the parameter is calculated by analyzing the image using automatic image-processing techniques. For example, dimensions of the vessel and/or the device may be calculated using techniques as described in US 2012/0230565 to Steinberg, and/or US 2010/0222671 to Cohen. Alternatively or additionally, functional parameters may be calculated automatically, for example, using techniques as described in WO 14/002095 to Tolkowsky.

For some applications, in order to calculate the parameter, vessel and/or device edge lines are automatically identified, using techniques described herein. For example, scan lines may be generated perpendicular to the centerline of a segment of the vessel that is sampled. The image is resampled along the scan lines. Corresponding gray-level values are stored as columns of a rectangular matrix M, thereby resampling the segment of the vessel as a straightened vessel segment. For the straightened vessel segment, optimum upper and lower paths are determined (with respect to the middle row of M), which connect the first and last columns of M. The optimization criterion takes into account the changes in gray-level along columns of M, and the paths' slopes. The vessel edge lines are obtained via back projection of upper and lower optimal paths on the original image.

A shortest path algorithm (e.g., as described in an article by Dijkstra, entitled "A Note on Two Problems in Connexion with Graphs" (Numerische Mathematik 1, 269-271, 1959)) is used in order to avoid irregularities, such as small gaps and loops, in the edge lines. For some applications, the centerline is corrected based upon the detected edge lines, and new scan lines are constructed. For each new scan line, vessel and/or device diameter is defined as a distance between the two points where the scan line intersects edge lines.

For some applications, the techniques described herein are performed with respect to other devices, and/or with respect to other portions of a subject's body to those described hereinabove.

For some applications, the techniques described herein are used in order to determine a parameter that relates to a hole-closure device. For example, the hole-closure device may be an atrial septal defect closure device, a left-atrial appendage closure device, and/or a hole-closure device that is used to close a surgically-created hole, such as in the apex of the subject's heart, and/or in a peripheral blood vessel, such as the femoral vein or the femoral artery. In response to determining the presence of a device within an image of a portion of the subject's body, and classifying the device as a hole-closure device, computer processor 28 may determine the maximum diameter of the hole-closure device, subsequent to the deployment of the hole-closure device. Alternatively or additionally, the techniques described herein may be used in order to determine a parameter that relates to an implantable valve (such as a prosthetic aortic valve, and/or a prosthetic mitral valve), e.g., the maximum diameter of the valve, subsequent to deployment of the valve. Further alternatively or additionally, the techniques described herein may be used in order to determine a parameter that relates to a blood vessel filter (e.g., a vena cava filter, such as the Crux® Vena Cava Filter, manufactured by Volcano Corporation (CA, USA)), e.g., the maximum diameter of the filter, subsequent to deployment of the filter within a blood vessel.

For some applications, the techniques described herein are used in order to determine a parameter that is related to a previously-implanted device (such as a stent, a graft or a replacement valve that was implanted prior to the present procedure being performed (e.g., at least one day prior to the present procedure being performed), in response to determining a presence of the previously-implanted device within an image of a portion of the subject's body, and in response to classifying the previously-implanted device as a given type of device.

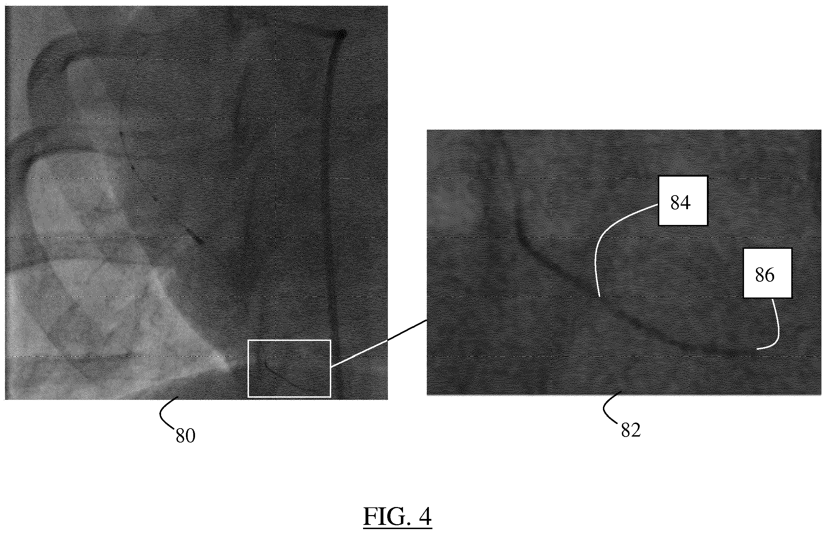

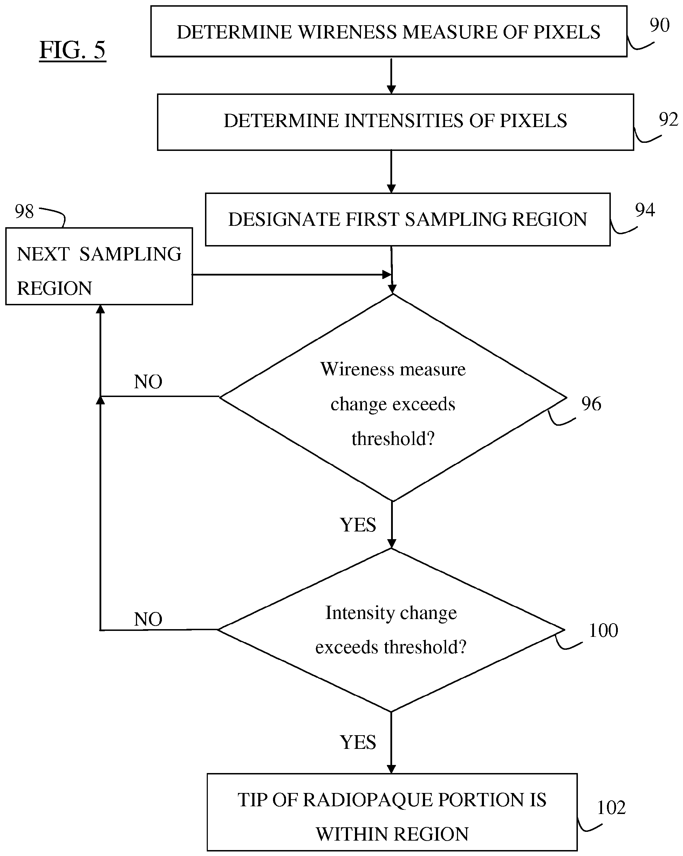

Reference is now made to Fig. 4, which is a schematic illustration of an image of a blood vessel, a wire (e.g., a guidewire) being disposed inside the blood vessel, in accordance with some applications of the present invention. Right frame 82 of Fig. 4A is an enlargement of a portion of left frame 80, the enlarged portion containing an image of a radiopaque end portion 84 of the guidewire. For some applications, a tip 86 of the radiopaque portion of the guidewire is automatically identified, using techniques described herein. Typically, as may be observed in Fig. 4, in a fluoroscopic image (or a different extraluminal image) of a blood vessel, there are darkened pixels within the image due to noise. Therefore, typically it is not possible to distinguish pixels that correspond to the radiopaque portion of the guidewire from surrounding pixels simply by analyzing the intensities of respective pixels. For some applications, computer processor 28 automatically determines a location of a tip of a radiopaque portion of the wire within the image using a technique as described with reference to Fig. 5.

Reference is now made to Fig. 5, which is a flowchart showing steps of a procedure that is performed by computer processor 28 in order to determine a location of a tip of a radiopaque portion of the wire within an extraluminal image of a blood vessel, in accordance with some applications of the present invention.

For each pixel within at least a portion of the image, a wireness measure of the pixel is determined (step 90). (It is noted that as used herein, the term "pixel" should not be interpreted to be limited to the smallest controllable element of the picture represented on the screen. Rather, as used herein, the term "pixel" should be interpreted to mean a set of one or more such elements.) For some applications, wireness measures are determined for pixels within the entire image. Alternatively, wireness measures of pixels within only a portion of the image are determined. For example, wireness measures of pixels within a region of the image in which the radiopaque portion of the wire is expected to be disposed (e.g., based on locations of other features within the image) may be sampled. Alternatively or additionally, the processor may receive an input from the user indicating a region of the image in which the radiopaque portion of the wire is expected to be disposed.

The wireness measure is a measure of the extent to which each of the pixels has a wire-like characteristic. For some applications, the wireness measure of the pixel is determined using one or more algorithms described herein. Typically, for each of the selected pixels, the wireness measure is determined, by measuring an extent to which the pixel, together with other pixels within the image, forms part of a long, thin set of pixels having a given characteristic, such as darkness, brightness, and/or a different characteristic (i.e., a wireness-indicating characteristic). Typically, the wireness measure is indicative of the pixel, together with the other pixels within the set, corresponding to the wire.

For some applications, generally similar techniques to those described in US 2012/0230565 to Steinberg, and/or US 2010/0222671 to Cohen, for determining a vesselness measure of a pixel are used for determining the wireness measure of a pixel. For example, wireness may be determined by means of a Hessian filter, such as the filter described in the article by Frangi et al., entitled "Multiscale vessel enhancement filtering" (Medical Image Computing and Computer Assisted Intervention--MICCAI 1998--Lecture Notes in Computer Science, vol. 1496, Springer Verlag, Berlin, Germany, pp. 130-137), and/or by means of a filter that performs enhancement and/or detection and/or segmentation of curvilinear structures. For some applications, a filter is used that is similar to a Frangi filter, but that differs from a Frangi filter (a) in that wireness is a homogeneous function, and/or (b) in the multipliers employed for the normalization of scales.

For some applications, the wireness measure of a pixel is obtained by determining the extent to which the gradient of the pixel is orthogonal to the eigenvector of the Hessian matrix corresponding to the highest eigenvalue. For some applications, the determination is assisted by a voting function applied to pixels that are adjacent to those pixels that are eventually determined to constitute the wire itself.

For some applications, thresholding is applied to image pixels by means of hysteresis. For example, pixels the wireness values of which fall below the high threshold of the hysteresis, but yet above the low threshold of the hysteresis, are incorporated into the set of pixels if they are contiguous with pixels that fall at or above the high threshold of the hysteresis.

For some applications, the pixels which form the aforementioned set are determined by means of morphological operations. For example, such morphological operations may include the skeletonization of a thresholded vesselness image. For some applications, the threshold applied is adaptive according to the specific region in the image.

For some applications, machine-learning techniques are used to determine wireness measures of the pixels.

In the next step of the procedure, for each pixel within at least the portion of the image, an intensity of the pixel is determined (step 92). It is noted that, for some applications, steps 90 and 92 are performed in the reverse order to that shown in the flowchart in Fig. 5.

Subsequent to the wireness measures and the intensities of the pixels within the portion of the image having been determined, computer processor 28 designates a first sampling region within the portion of the image (step 94). For some applications, the first sampling region is designated in accordance with one or more algorithms described herein. For example, the sampling region may include a single pixel, or a plurality of pixels. The first sampling region may be generated randomly, and/or in response to an input from a user. For some applications, the processor designates the first sampling region by designating a sampling region in which the tip of the guidewire is likely to be disposed. For example, the processor may designate the first sampling region by designating one or more regions that have high values of the wireness measure. Alternatively or additionally, the processor may designate the first sampling region in response to determining that a region of the image is likely to contain the tip of the wire, based upon machine-learning analysis of the image.

For some applications, step 94 is performed before steps 90 and 92, and steps 90 and 92 are only performed on the designated sampling region.

The processor determines whether, within the first sampling region, there is at least one pixel, at which there is a change in the wireness measure, by more than a threshold amount, relative to the value of the wireness measure of one or more pixels that are adjacent to the pixel and that have the wireness-indicating characteristic (step 96). For some applications, the processor performs step 96 using one or more algorithms described herein. For example, by way of illustration, the processor may determine that from one pixel to an adjacent pixel there is a decrease in the wireness measure that exceeds a threshold percentage decrease. Or, the processor may determine that at least one pixel has a wireness measure that is lower than the mean wireness measure of all of the pixels belonging to the set of pixels, by more than a threshold percentage, whereas one or more pixels that are adjacent to the pixel have wireness measure(s) that exceeds the threshold.

For some applications, within the designated region, the processor determines the local directionality of the set of pixels that have the wireness-indicating characteristic. The processor determines, along that direction, whether there is at least one pixel at which there is a change in the wireness measure by more than a threshold amount, relative to one or more pixels that are adjacent to the pixel and that have the wireness-indicating characteristic.

In response to determining that there is not a change in the wireness measure at at least one pixel that exceeds the threshold, within the first sampling region, the processor proceeds to the next sampling region (step 98), and step 96 is repeated at the second sampling region. The second sampling region is typically selected in a generally similar manner to the selection of the first sampling region, and/or based upon a spatial relationship to the first sampling region. In response to determining that the change in the wireness measure at at least one pixel within a sampling region does exceed the threshold, the processor then determines whether, at the at least one pixel there is a change, by more than a threshold amount, in the intensity of the pixel relative to the value of the intensity of at least some of the set of pixels having the wireness-indicating characteristic (step 100). For some applications, the processor performs step 100 using one or more algorithms described herein. For example, by way of illustration, the processor may determine that from one of the pixels to an adjacent pixel there is an increase in intensity that exceeds a threshold percentage increase. Or, the processor may determine that one of the pixels has an intensity that exceeds the mean intensity of all of the pixels belonging to the set of pixels, by more than a threshold percentage.

For some applications, within the designated region, the processor determines the local directionality of the set of pixels that have the wireness-indicating characteristic. The processor determines, along that direction, whether there is at least one pixel at which there is a change in intensity by more than a threshold amount, relative to at least some of the pixels that belong to the set of pixels that have the wireness-indicating characteristic.

In response to determining that there is not a change in the intensity at at least one pixel that exceeds the threshold, within the current sampling region, the processor proceeds to the next sampling region (step 98), and step 96 is repeated at the next sampling region. In response to determining that the change in intensity at at least one pixel within the current sampling region does exceed the threshold, it is determined that the tip of the radiopaque portion of the wire is disposed within the current sampling region (step 102). An output is generated in response to the determined location of the tip of the radiopaque portion of the wire within the image.

It is noted that, for some applications, steps 96 and 100 are performed in the reverse order, the processor first determining whether there is a change in the intensity at at least one pixel by more than a threshold amount, and, subsequently, determining whether there is a change in the wireness measure at the at least one pixel by more than the threshold amount.

Typically, an output is generated by computer processor 28 in response to the determined location of the tip of the radiopaque portion of the wire within the image. For some applications, the processor determines locations of both of the tips of the radiopaque portion of the wire, and thereby determines the location of the radiopaque portion of the wire, and/or the center of the radiopaque portion of the wire within the image. The output is generated in response to the determined location of the radiopaque portion of the wire, and/or in response to the determined location of the center of the radiopaque portion of the wire within the image.

For some applications, in response to determining the location of the tip of the radiopaque portion of the wire within a given image, the processor aligns the image with a second image, by aligning the radiopaque portions of the wires in each of the images with one another. In accordance with respective applications, the aligned images may be displayed in an image stream in which the images are aligned with one another, and/or a composite image may be generated based upon the alignment of the image and the second image. For example, the processor may average the image and the second image, subsequent to aligning the images with one another. In general, the identification of the location of the tip of the radiopaque portion of the wire within a given image may be used to perform any of the image stabilization and/or enhancement techniques that are described in any one of US 2008/0221440 to Iddan, US 2012/0230565 to Steinberg, and US 2010/0222671 to Cohen.

For some applications, identification of the location of the tip of the radiopaque portion of the wire within a given image may be used to facilitate the determination of the location of an endoluminal device within the lumen, for example, in accordance with techniques described in US 2012/0004537 to Tolkowsky, and/or WO 13/174472 Steinberg. For example, the location within the blood vessel at which one or more endoluminal data points were acquired by the endoluminal data-acquisition device (e.g., an endoluminal imaging device, or an endoluminal data-acquisition device that is configured to acquire a plurality of functional endoluminal data points) may be determined. Based upon the determined locations within the blood vessel at which the endoluminal data points were acquired, the processor may generate an output, such as by generating an endoluminal imaging stack, and/or by generating an indication of the correspondence between an endoluminal data point and the location within the blood vessel at which the endoluminal data point was acquired.

For some applications, endoluminal data points are acquired by positioning an endoluminal data-acquisition device along a luminal segment of the blood vessel that includes a designated luminal site. Subsequently, while observing extraluminal images of the luminal segment, one or more locations along that segment are indicated by a user input device (e.g., user interface 30). In response to the indication of the one or more locations by the user input device, the corresponding, previously-acquired endoluminal images are displayed.

Typically, the designated luminal site includes a site being diagnosed, at which, subject to the outcome of the diagnosis, a therapeutic device will be positioned and deployed, e.g., the site of an anatomical feature, the implantation site of a previously-implanted device, and/or a site at a defined location with respect to the implantation site. For example, the designated luminal site may include a portion of the lumen that is narrow with respect to surrounding portions of the lumen, and/or the site of a lesion.

For some applications, endoluminal data points are acquired by positioning an endoluminal data-acquisition device at a designated luminal site. Subsequently, an endoluminal therapeutic device is positioned and deployed at the designated luminal site under extraluminal imaging, while concurrently viewing on-line the endoluminal data that were previously acquired by the endoluminal data-acquisition device at the current location of the therapeutic device. Typically, endoluminal data are acquired at respective endoluminal sites in the vicinity of the designated endoluminal site. Subsequently, when the endoluminal therapeutic device is placed inside the lumen, previously-acquired endoluminal data are displayed and updated, typically automatically and typically on-line, to correspond to the current location of the therapeutic device (or of a portion thereof), the location of the therapeutic device typically changing during the positioning of the therapeutic device.

For some applications, extraluminal imaging and the previously-acquired endoluminal data points are co-used such that it is as if the therapeutic device is being positioned and deployed under both real-time extraluminal imaging and real-time endoluminal data acquisition. This is because (a) the extraluminal imaging is performed in real-time, and (b), although the endoluminal data are not acquired in real-time, endoluminal data are displayed that correspond to the current location of the therapeutic device.

In accordance with some applications of the present invention, when the therapeutic device is disposed inside the lumen, the location of the device within the lumen is determined by performing image processing on the extraluminal image of the device inside the lumen.

For some applications, identification of the location of the tip of the radiopaque portion of the wire within a given image may be used to facilitate the determination of a transformation function for mapping between the image and a second image, for example, in accordance with techniques described in WO 13/174472 to Steinberg. For example, a transformation function may be determined for mapping a current fluoroscopic image to a previously acquired angiographic image, or vice versa. For some applications, a transformation function is determined for mapping a current fluoroscopic image to a previously acquired angiographic image, by comparing an arrangement of two or more features within the current fluoroscopic image to a shape of at least a portion of a pathway within the previously acquired angiographic image. For some applications, at least one of the features is the radiopaque portion of the guidewire in the current fluoroscopic image.

For some applications, based upon the determined transformation function, the processor determines the location of an endoluminal device within the lumen, for example, in accordance with techniques described in WO 13/174472 to Steinberg. For example, the location within the blood vessel at which one or more endoluminal data points were acquired by the endoluminal data-acquisition device (e.g., an endoluminal imaging device, or an endoluminal data-acquisition device that is configured to acquire a plurality of functional endoluminal data points) may be determined. Based upon the determined locations within the blood vessel at which the endoluminal data points were acquired, the processor may generate an output, such as by generating an endoluminal imaging stack, and/or by generating an indication of the correspondence between an endoluminal data point and the location within the blood vessel at which the endoluminal data point was acquired. Alternatively, based upon the determined location within the blood vessel at which the endoluminal data point was acquired, the processor may co-use endoluminal data points and extraluminal imaging using techniques described hereinabove, and/or as described in US 2012/0004537 to Tolkowsky, and/or WO 13/174472 to Steinberg.

For some applications, identification of the location of the tip of the radiopaque portion of the wire within a given image may be used to facilitate the classification of a portion of the image as being associated with the distal end of a guidewire, for example, in accordance with techniques described in WO 13/174472 to Steinberg. For example, classification of a portion of the image as being associated with the distal end of a guidewire may be used to facilitate the determination of a transformation function for mapping one image to another image, in accordance with techniques described in WO 13/174472 to Steinberg.

It is noted that although some techniques described herein are described primarily with respect to extraluminal fluoroscopic/angiographic images and endoluminal images, the scope of the present invention includes applying the techniques described herein to other forms of extraluminal and endoluminal images and/or data, *mutatis mutandis.* For example, the extraluminal images may include images generated by fluoroscopy, CT, MRI, ultrasound, PET, SPECT, other extraluminal imaging techniques, or any combination thereof. Endoluminal images may include images generated by intravascular ultrasound (IVUS) optical coherence tomography (OCT), near-infrared spectroscopy (NIRS), intravascular ultrasound (IVUS), endobronchial ultrasound (EBUS), magnetic resonance (MR), other endoluminal imaging techniques, or any combination thereof. Endoluminal data may include data related to pressure (e.g., fractional flow reserve), flow, temperature, electrical activity, or any combination thereof.

Although some techniques described herein are described primarily as being performed on a blood vessel, the scope of the present application includes performing similar techniques on a lumen in the vascular system, the respiratory tract, the digestive tract, the urinary tract, any other luminal structure within a patient's body, or any other suitable anatomical structure within a patient's body, *mutatis mutandis.* Examples of an anatomical structure to which the techniques described herein may be applied include a coronary vessel, a coronary lesion, a vessel, a vascular lesion, a lumen, a luminal lesion, and/or a valve.

Applications of the invention described herein can take the form of a computer program product accessible from a computer-usable or computer-readable medium providing program code for use by or in connection with a computer or any instruction execution system, such as computer processor 28. For the purposes of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk-read only memory (CD-ROM), compact disk-read/write (CD-R/W) and DVD.

A data processing system suitable for storing and/or executing program code will include at least one processor (e.g., computer processor 28) coupled directly or indirectly to memory elements (e.g., memory 29) through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object oriented programming language such as Java, Smalltalk, C++ or the like and conventional procedural programming languages, such as the C programming language or similar programming languages.

It will be understood that each block of the flowcharts shown in Figs. 3A, 3B, 3C, and 5, and combinations of blocks in the flowchart, can be implemented by computer program instructions. These computer program instructions may be provided to a processor of a general purpose computer, special purpose computer, or other programmable data processing apparatus to produce a machine, such that the instructions, which execute via the processor of the computer (e.g., computer processor 28) or other programmable data processing apparatus, create means for implementing the functions/acts specified in the flowcharts and/or algorithms described in the present application. These computer program instructions may also be stored in a computer-readable medium that can direct a computer or other programmable data processing apparatus to function in a particular manner, such that the instructions stored in the computer-readable medium produce an article of manufacture including instruction means which implement the function/act specified in the flowchart blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the flowcharts and/or algorithms described in the present application.

Computer processor 28 is typically a hardware device programmed with computer program instructions to produce a special purpose computer. For example, when programmed to perform the algorithms described with reference to Figs. 2A-C and 3A-C, computer processor 28 typically acts as a special purpose device-specific parameter measurement computer processor. When programmed to perform the algorithms described with reference to Figs. 4 and 5, computer processor 28 typically acts as a special purpose guidewire-tip-identifying computer processor. Typically, the operations described herein that are performed by computer processor 28 transform the physical state of memory 29, which is a real physical article, to have a different magnetic polarity, electrical charge, or the like depending on the technology of the memory that is used.

The scope of the present application includes combining the apparatus and methods described herein with apparatus and methods described in any one of the following applications:
- International Application PCT/IL2008/000316 to Iddan (published as WO 08/107905), filed March 09, 2008, entitled "Imaging and tools for use with moving organs."
- US Patent Application 12/075,252 to Iddan (published as US 2008/0221440), filed March 10, 2008, entitled "Imaging and tools for use with moving organs;"
- International Application PCT/IL2009/000610 to Iddan (published as WO 09/153794), filed June 18,2009, entitled "Stepwise advancement of a medical tool;"
- US Patent Application 12/487,315 to Iddan (published as US 2009/0306547), filed June 18, 2009, entitled "Stepwise advancement of a medical tool;"
- US Patent Application 12/666,879 to Steinberg (published as US 2012/0230565), which is the US national phase of PCT Application No. PCT/IL2009/001089 to Cohen (published as WO 10/058398), filed November 18, 2009, entitled "Image processing and tool actuation for medical procedures;"
- US Patent Application 12/781,366 to Cohen (published as US 2010/0222671), filed May 17, 2010, entitled "Identification and presentation of device-to-vessel relative motion;"
- International Patent Application PCT/IL2011/000391 (published as WO 11/145094), entitled "Identification and presentation of device-to-vessel relative motion," filed May 17, 2011;
- US 13/228,229 to Tolkowsky (published as US 2012/0004537), filed September 08, 2011, which is a continuation of International Application No. PCT/IL2011/000612 to Tolkowsky (published as WO 12/014212), filed 28 July, 2011 entitled "Co-use of endoluminal data and extraluminal imaging;"
- US Patent Application 14/128,243 (published as US 2014/0140597), which is the US national phase of International Patent Application PCT/IL2012/000246 (published as WO 12/176191), filed June 21, 2012, entitled "Luminal background cleaning;"
- US Patent Application 13/228,229 to Tolkowsky (published as US 2012/0004537), entitled "Co-use of endoluminal data and extraluminal imaging," filed September 08, 2011;
- US Patent Application 14/097,922 to Steinberg (published as US 2014/0094691), filed December 05, 2013, entitled "Co-use of endoluminal data and extraluminal imaging," which is a continuation of International Application PCT/IL2013/050438 (published as WO 13/174472) to Steinberg, filed May 21, 2013, entitled "Co-use of endoluminal data and extraluminal imaging;" and
- US Patent Application 14/142,082 to Tolkowsky (published as US 2014/0121513), filed December 27, 2013, entitled "Determining a characteristic of a lumen by measuring velocity of a contrast agent," which is a continuation of International Application PCT/IL2013/050549 (published as WO 14/002095) to Tolkowsky, filed June 26, 2013, entitled "Flow-related image processing in luminal organs."

It will be appreciated by persons skilled in the art that the present invention is not limited to what has been particularly shown and described hereinabove. Rather, the scope of the present invention includes both combinations and subcombinations defined by the appended claims.

## Claims

1. Apparatus for use with an image of a wire within a blood vessel of a subject, the wire including a radiopaque portion, the apparatus comprising:
an output device; and
at least one computer processor configured:
for each pixel within at least a portion of the image, to determine a wireness measure of the pixel and an intensity of the pixel, wherein the wireness measure of the pixel is a measure of an extent to which the pixel has a wire-like characteristic and is determined by measuring an extent to which the pixel, together with other pixels within the image, forms part of a long, thin set of pixels having a wireness-indicating characteristic;
to determine that the tip of the radiopaque portion of the wire is located within a region within the portion of the image, by detecting that, within the region, there is at least one pixel at which there is a change, by more than respective threshold amounts, in both
the wireness measure of the pixel relative to the wireness measure of one or more adjacent pixels with wireness-indicating characteristic and
the intensity of the pixel relative to the intensity of at least some of the pixels with wireness-indicating characteristic; and
to generate an output on the output device, in response to the determined location of the tip of the radiopaque portion of the wire within the image.

2. The apparatus according to claim 1,
wherein the computer processor is configured to determine a location of the radiopaque portion of the wire within the image, based upon the determined location of the tip of the radiopaque portion of the wire, and
wherein the processor is configured to generate the output in response to the determined location of the radiopaque portion of the wire within the image.

3. The apparatus according to claim 1,
wherein the computer processor is configured to determine a location of a center of the radiopaque portion of the wire within the image, based upon the determined location of the tip of the radiopaque portion of the wire, and
wherein the computer processor is configured to generate the output in response to the determined location of the center of the radiopaque portion of the wire within the image.

4. The apparatus according to claim 1, wherein the computer processor is configured to determine the wireness measure for each of the pixels within at least the portion of the image using machine-learning techniques.

5. The apparatus according to claim 1, wherein the computer processor is configured to determine the wireness measure for each of the pixels within at least the portion of the image by measuring, for each of the pixels, an extent to which the pixel, together with other pixels within the image, forms part of a continuous long, thin set of pixels having a given characteristic.

6. The apparatus according to claim 1, wherein the computer processor is configured to determine the wireness measure for each of the pixels within at least the portion of the image either by analyzing eigenvalues of a multiscale second order local structure of at least the portion of the image or by applying a filter that detects curvilinear structures to at least the portion of the image.

7. The apparatus according to claim 1, wherein the computer processor is configured to determine the wireness measure for each of the pixels within at least the portion of the image either by applying a filter that enhances curvilinear structures to at least the portion of the image or by applying a filter that segments curvilinear structures to at least the portion of the image.

8. The apparatus according to any one of claims 1-7, wherein the computer processor is configured to align the image with a second image, based upon the determined location of the tip of the radiopaque portion of the wire within the image.

9. The apparatus according to claim 8, wherein the computer processor is configured to generate the output by displaying the image and the second image in an image stream in which the image and the second image are aligned with one another.

10. The apparatus according to claim 8, wherein the computer processor is configured to generate the output by generating a composite image, based upon the alignment of the image and the second image.

11. The apparatus according to any one of claims 1-7, wherein the computer processor is configured to determine a transformation function for mapping between the image and a second image at least partially in response to the determined location of the wire within the image, and wherein the computer processor is configured to generate the output based upon the determined transformation function.

12. The apparatus according to claim 11, wherein the computer processor is configured, based upon the determined transformation function, to determine a location of an endoluminal device within the blood vessel, and wherein the computer processor is configured to generate the output in response to the determined location of the endoluminal device.

13. The apparatus according to claim 12, wherein:
the endoluminal device includes an endoluminal data-acquisition device,
the computer processor is configured to determine the location of the endoluminal device within the blood vessel by determining a location within the blood vessel at which an endoluminal data point was acquired by the endoluminal data-acquisition device, and
the computer processor is configured to generate the output based upon determining the location within the blood vessel at which the endoluminal data point was acquired by the endoluminal data-acquisition device.

14. The apparatus according to any one of claims 1-7, wherein the computer processor is configured, based upon the determined location of the wire within the image, to determine a location of an endoluminal device within the blood vessel, and wherein the computer processor is configured to generate the output in response to the determined location of the endoluminal device.

15. The apparatus according to claim 14, wherein:
the endoluminal device includes an endoluminal data-acquisition device,
the computer processor is configured to determine the location of the endoluminal device within the blood vessel by determining a location within the blood vessel at which an endoluminal data point was acquired by the endoluminal data-acquisition device, and
the computer processor is configured to generate the output based upon determining the location within the blood vessel at which the endoluminal data point was acquired by the endoluminal data-acquisition device.

## Patentansprüche

1. Vorrichtung zur Verwendung mit einem Bild eines Drahts innerhalb eines Blutgefäßes eines Subjekts, wobei der Draht einen röntgendichten Abschnitt enthält, die Vorrichtung umfassend:
eine Ausgabevorrichtung; und
zumindest einen Computerprozessor, konfiguriert:
für jedes Pixel innerhalb zumindest eines Abschnitts des Bildes ein Drahtformmaß des Pixels und eine Intensität des Pixels zu bestimmen, wobei das Drahtformmaß des Pixels ein Maß eines Ausmaßes ist, in dem das Pixel eine drahtförmige Eigenschaft hat, und durch Messen eines Ausmaßes bestimmt wird, in dem das Pixel, gemeinsam mit anderen Pixeln innerhalb des Bildes, einen langen dünnen Satz von Pixeln mit Drahtform angebender Eigenschaft bildet;
zu bestimmen, dass die Spitze des röntgendichten Abschnitts des Drahts innerhalb einer Region innerhalb des Abschnitts des Bildes gelegen ist, indem detektiert wird, dass innerhalb der Region zumindest ein Pixel vorhanden ist, an dem eine Änderung um mehr als einen jeweiligen Schwellenbetrag sowohl
im Drahtformmaß des Pixels relativ zum Drahtformmaß eines oder mehrerer benachbarter Pixel mit Drahtform angebender Eigenschaft wie auch
in der Intensität des Pixels relativ zur Intensität zumindest einiger der Pixel mit Drahtform angebender Eigenschaft vorliegt; und
einen Ausgang der Ausgabevorrichtung in Antwort auf die bestimmte Stelle der Spitze des röntgendichten Abschnitts des Drahts innerhalb des Bildes zu generieren.

2. Vorrichtung nach Anspruch 1,
wobei der Computerprozessor konfiguriert ist, eine Stelle des röntgendichten Abschnitts des Drahts innerhalb des Bildes auf Basis der bestimmten Stelle des röntgendichten Abschnitts des Drahts zu bestimmen, und
wobei der Prozessor konfiguriert ist, den Ausgang in Antwort auf die bestimmte Stelle des röntgendichten Abschnitts des Drahts innerhalb des Bildes zu generieren.

3. Vorrichtung nach Anspruch 1,
wobei der Computerprozessor konfiguriert ist, eine Stelle eines Mittelpunkts des röntgendichten Abschnitts des Drahts innerhalb des Bildes auf Basis der bestimmten Stelle des röntgendichten Abschnitts des Drahts zu bestimmen, und
wobei der Computerprozessor konfiguriert ist, den Ausgang in Antwort auf die bestimmte Stelle des Mittelpunkts des röntgendichten Abschnitts des Drahts innerhalb des Bildes zu generieren.

4. Vorrichtung nach Anspruch 1, wobei der Computerprozessor konfiguriert ist, das Drahtformmaß für jedes der Pixel innerhalb zumindest des Abschnitts des Bildes mittels Maschinenlemtechniken zu bestimmen.

5. Vorrichtung nach Anspruch 1, wobei der Computerprozessor konfiguriert ist, das Drahtformmaß für jedes der Pixel innerhalb zumindest des Abschnitts des Bildes durch Messen, für jedes der Pixel, eines Ausmaßes, in dem das Pixel gemeinsam mit anderen Pixeln innerhalb des Bildes einen kontinuierlichen, langen, dünnen Satz von Pixeln mit einer gegebenen Eigenschaft bildet.

6. Vorrichtung nach Anspruch 1, wobei der Computerprozessor konfiguriert ist, das Drahtformmaß für jedes der Pixel innerhalb zumindest des Abschnitts des Bildes entweder durch Analysieren von Eigenwerten einer örtlichen Mehrskalenstruktur zweiter Ordnung zumindest des Abschnitts des Bildes oder durch Anwenden eines Filters, der kurvenförmige Strukturen erfasst, an zumindest dem Abschnitt des Bildes zu bestimmen.

7. Vorrichtung nach Anspruch 1, wobei der Computerprozessor konfiguriert ist, das Drahtformmaß für jedes der Pixel innerhalb zumindest des Abschnitts des Bildes entweder durch Anwenden eines Filters, der kurvenförmige Strukturen verstärkt, an zumindest dem Abschnitt des Bildes oder durch Anwenden eines Filters, der kurvenförmige Strukturen segmentiert, an zumindest dem Abschnitt des Bildes zu bestimmen.

8. Vorrichtung nach einem der Ansprüche 1-7, wobei der Computerprozessor konfiguriert ist, das Bild mit einem zweiten Bild auf Basis der bestimmten Stelle der Spitze des röntgendichten Abschnitts des Drahts innerhalb des Bildes auszurichten.

9. Vorrichtung nach Anspruch 8, wobei der Computerprozessor konfiguriert ist, den Ausgang durch Anzeigen des Bildes und des zweiten Bildes in einem Bildstrom zu generieren, in dem das Bild und das zweite Bild miteinander ausgerichtet sind.

10. Vorrichtung nach Anspruch 8, wobei der Computerprozessor konfiguriert ist, den Ausgang durch Generieren eines zusammengesetzten Bildes auf Basis der Ausrichtung des Bildes und des zweiten Bildes zu generieren.

11. Vorrichtung nach einem der Ansprüche 1-7, wobei der Computerprozessor konfiguriert ist, eine Transformationsfunktion zur Abbildung zwischen dem Bild und einem zweiten Bild zumindest teilweise in Antwort auf die bestimmte Stelle des Drahts innerhalb des Bildes zu bestimmen, und wobei der Computerprozessor konfiguriert ist, den Ausgang auf Basis der bestimmten Transformationsfunktion zu generieren.

12. Vorrichtung nach Anspruch 11, wobei der Computerprozessor konfiguriert ist, auf Basis der bestimmten Transformationsfunktion eines Stelle einer endoluminalen Einrichtung innerhalb des Blutgefäßes zu bestimmen, und wobei der Computerprozessor konfiguriert ist, den Ausgang in Antwort auf die bestimmte Stelle der endoluminalen Einrichtung zu generieren.

13. Vorrichtung nach Anspruch 12, wobei:
die endoluminale Einrichtung eine endoluminale Datenerlangungseinrichtung enthält,
der Computerprozessor konfiguriert ist, die Stelle der endoluminalen Einrichtung innerhalb des Blutgefäßes durch Bestimmen einer Stelle innerhalb des Blutgefäßes zu bestimmen, an der ein endoluminaler Datenpunkt durch die endoluminale Datenerlangungseinrichtung erlangt wurde, und
der Computerprozessor konfiguriert ist, den Ausgang auf Basis einer Bestimmung der Stelle innerhalb des Blutgefäßes, an der der endoluminale Datenpunkt durch die endoluminale Datenerlangungseinrichtung erlangt wurde, zu generieren.

14. Vorrichtung nach einem der Ansprüche 1-7, wobei der Computerprozessor konfiguriert ist, auf Basis der bestimmten Stelle des Drahts innerhalb des Bildes eine Stelle einer endoluminalen Einrichtung innerhalb des Blutgefäßes zu bestimmen, und wobei der Computerprozessor konfiguriert ist, den Ausgang in Antwort auf die bestimmte Stelle der endoluminalen Einrichtung zu generieren.

15. Vorrichtung nach Anspruch 14, wobei:
die endoluminale Einrichtung eine endoluminale Datenerlangungseinrichtung enthält,
der Computerprozessor konfiguriert ist, die Stelle der endoluminalen Einrichtung innerhalb des Blutgefäßes durch Bestimmen einer Stelle innerhalb des Blutgefäßes zu bestimmen, an der ein endoluminaler Datenpunkt durch die endoluminale Datenerlangungseinrichtung erlangt wurde, und
der Computerprozessor konfiguriert ist, den Ausgang auf Basis einer Bestimmung der Stelle innerhalb des Blutgefäßes, an der der endoluminale Datenpunkt durch die endoluminale Datenerlangungseinrichtung erlangt wurde, zu generieren.

## Revendications

1. Appareil pour utilisation avec une image d'un fil métallique dans un vaisseau sanguin d'un sujet, le fil métallique comprenant une partie radio-opaque, l'appareil comprenant :
un dispositif de sortie ; et
au moins un processeur d'ordinateur configuré,
pour chaque pixel dans au moins une partie de l'image, pour déterminer une mesure de l'aspect filaire du pixel et une intensité du pixel, dans lequel la mesure de l'aspect filaire du pixel est une mesure de l'ampleur avec laquelle le pixel a une caractéristique de type filaire et est déterminée en mesurant l'ampleur avec laquelle le pixel, conjointement avec d'autres pixels au sein de l'image, fait partie d'un ensemble long et mince de pixels ayant une caractéristique indicatrice d'aspect filaire ;
pour déterminer que la pointe de la partie radio-opaque du fil métallique est située dans une région dans la partie de l'image en détectant que, au sein de la région, il y a au moins un pixel dans lequel il y a un changement de plus de quantités de seuil respectives, à la fois dans la mesure de l'aspect filaire du pixel par rapport à la mesure de l'aspect filaire d'un ou plusieurs pixels adjacents avec une caractéristique indicatrice d'aspect filaire ; et
l'intensité du pixel par rapport à l'intensité d'au moins certains des pixels avec une caractéristique indicatrice d'aspect filaire ; et
pour générer une sortie sur le dispositif de sortie en réponse à l'emplacement déterminé de la pointe de la partie radio-opaque du fil métallique dans l'image.

2. Appareil selon la revendication 1,
dans lequel le processeur informatique est configuré pour déterminer un emplacement de la partie radio-opaque du fil dans l'image sur la base de l'emplacement déterminé de la pointe de la partie radio-opaque du fil et
dans lequel le processeur est configuré pour générer la sortie en réponse à l'emplacement déterminé de la partie radio-opaque du fil dans l'image.

3. Appareil selon la revendication 1,
dans lequel le processeur informatique est configuré pour déterminer un emplacement d'un centre de la partie radio-opaque du fil dans l'image sur la base de l'emplacement déterminé de la pointe de la partie radio-opaque du fil et
dans lequel le processeur informatique est configuré pour générer la sortie en réponse à l'emplacement déterminé du centre de la partie radio-opaque du fil dans l'image.

4. Appareil selon la revendication 1, dans lequel le processeur informatique est configuré pour détermine la mesure de l'aspect filaire pour chacun des pixels dans au moins la partie de l'image en utilisant des techniques d'apprentissage sur machine.

5. Appareil selon la revendication 1, dans lequel le processeur informatique est configuré pour déterminer la mesure de l'aspect filaire pour chacun des pixels à l'intérieur d'au moins la partie de l'image en mesurant, pour chacun des pixels, l'ampleur avec laquelle le pixel, conjointement avec d'autres pixels au sein de l'image, fait partie d'un ensemble continu long et mince de pixels ayant une caractéristique donnée.

6. Appareil selon la revendication 1, dans lequel le processeur informatique est configuré pour déterminer la mesure de l'aspect filaire pour chacun des pixels dans au moins la partie de l'image par analyse de valeurs propres d'une structure locale du second ordre à échelles multiples d'au moins la partie de l'image en appliquant un filtre qui détecte des structures curvilignes sur au moins la partie de l'image.

7. Appareil selon la revendication 1, dans lequel le processeur informatique est configuré pour déterminer la mesure de l'aspect filaire pour chacun des pixels dans au moins la partie de l'image en appliquant un filtre qui renforce les structures curvilignes sur au moins la partie de l'image ou en appliquant un filtre qui segmente les structures curvilignes sur au moins la partie de l'image.

8. Appareil selon l'une quelconque des revendications 1 à 7, dans lequel le processeur informatique est configuré pour aligner l'image sur une seconde image sur la base de l'emplacement déterminé de la pointe de la partie radio-opaque du fil dans l'image.

9. Appareil selon la revendication 8, dans lequel le processeur informatique est configuré pour générer la sortie par affichage de l'image et de la seconde image dans un flux d'images dans lequel l'image et la seconde image sont alignées l'une avec l'autre.

10. Appareil selon la revendication 8, dans lequel le processeur informatique est configuré pour générer la sortie en générant une image composite sur la base de l'alignement de l'image et de la seconde image.

11. Appareil selon l'une quelconque des revendications 1 à 7, dans lequel le processeur informatique est configuré pour déterminer une fonction de transformation pour le mappage entre l'image et une seconde image au moins en partie en réponse à l'emplacement déterminé du fil dans l'image et dans lequel le processeur informatique est configuré pour générer la sortie sur la base de la fonction de transformation déterminée.

12. Appareil selon la revendication 11, dans lequel le processeur informatique est configuré sur la base de la fonction de transformation déterminée pour déterminer un emplacement d'un dispositif endoluminal dans le vaisseau sanguin et dans lequel le processeur informatique est configuré pour générer la sortie en réponse à l'emplacement déterminé du dispositif endoluminal.

13. Appareil selon la revendication 12, dans lequel :
le dispositif endoluminal comprend un dispositif d'acquisition de données endoluminales,
le processeur informatique est configuré pour déterminer l'emplacement du dispositif endoluminal dans le vaisseau sanguin en déterminant un emplacement dans le vaisseau sanguin où un point de données endoluminales a été acquis par le dispositif d'acquisition de données endoluminales et
le processeur informatique est configuré pour générer la sortie sur la base de la détermination de l'emplacement dans le vaisseau sanguin où le point de données endoluminales a été acquis par le dispositif d'acquisition de données endoluminales.

14. Appareil selon l'une quelconque des revendications 1 à 7, dans lequel le processeur informatique est configuré, sur la base de l'emplacement déterminé du fil dans l'image, pour déterminer un emplacement d'un dispositif endoluminal au sein du vaisseau sanguin et dans lequel le processeur informatique est configuré pour générer la sortie en réponse à l'emplacement déterminé du dispositif endoluminal.

15. Appareil selon la revendication 14, dans lequel :
le dispositif endoluminal comprend un dispositif d'acquisition de données endoluminales,
le processeur informatique est configuré pour déterminer l'emplacement du dispositif endoluminal dans le vaisseau sanguin en déterminant un emplacement dans le vaisseau sanguin où un point de données endoluminales a été acquis par le dispositif d'acquisition de données endoluminales et
le processeur informatique est configuré pour générer la sortie sur la base de la détermination de l'emplacement dans le vaisseau sanguin où le point de données endoluminales a été acquis par le dispositif d'acquisition de données endoluminales.
